(19) Europäisches Patentamt
European Patent Office
Office européen des brevets

(11) Veröffentlichungsnummer: **0 013 755**
**B1**

(12) **EUROPÄISCHE PATENTSCHRIFT**

(45) Veröffentlichungstag der Patentschrift: **22.06.83**

(51) Int. Cl.³: **C 08 L 91/06**, A 61 K 7/00

(21) Anmeldenummer: **79105344.0**

(22) Anmeldetag: **21.12.79**

# (54) Bienenwachsersatz auf der Basis langkettiger Fettsäureglyceride und dessen Verwendung.

(30) Priorität: **27.12.78 DE 2856277**

(43) Veröffentlichungstag der Anmeldung:
**06.08.80 Patentblatt 80/16**

(45) Bekanntmachung des Hinweises auf die Patenterteilung:
**22.06.83 Patentblatt 83/25**

(84) Benannte Vertragsstaaten:
**FR GB IT NL**

(56) Entgegenhaltungen:
**US - D - 303 702**

**Die Akte enthält technische Angaben, die nach dem Eingang der Anmeldung eingereicht wurden und die nicht in dieser Patentschrift enthalten sind.**

(73) Patentinhaber: **Henkel Kommanditgesellschaft auf Aktien**
**Postfach 1100 Henkelstrasse 67**
**D-4000 Düsseldorf 1 (DE)**

(72) Erfinder: **Scheuffgen, Ingeborg**
**Spulgasse 3**
**D-4040 Neuss (DE)**
Erfinder: **Ploog, Uwe, Dr.**
**Haydnweg 6**
**D-5657 Haan (DE)**

**EP 0 013 755 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Bienenwachsersatz auf der Basis langkettiger Fettsäureglyceride und dessen Verwendung

Gegenstand der Erfindung ist eine neue, als Ersatz für Bienenwachs auf dem kosmetischen Sektor dienende Komposition aus Mono-, Di- und Triglyceriden langkettiger Fettsäuren, Estern langkettiger Fettsäuren mit höhermolekularen Alkoholen, höheren Fettsäuren, höhermolekularen $\alpha$-verzweigten aliphatischen Monocarbonsäuren und gegebenenfalls mikrokristallinen Paraffinen.

Bienenwachs hat aufgrund seiner in mannigfacher Hinsicht günstigen Eigenschaften in der Kosmetik eine vielseitige Verwendung in Cremes, Schminken, Lippenstiften, Emulsionen, Salben, Depilatorien und dergleichen gefunden. Die Verknappung an Bienenwachs und die damit einhergehende Streckung und Verfälschung, die zu starken Qualitätsunterschieden führt, hat den Wunsch nach einem gleichwertigen Ersatz aufkommen lassen. Die Ergebnisse der bisherigen Bemühungen haben immer nur einem Teil der Bienenwachseigenschaften gerecht werden können und so ist die Forderung nach einem Ersatzprodukt, das dem Bienenwachs in seiner Gesamtheit möglichst nahe kommt, bestehen geblieben.

Aus der amerikanischen Patentschrift US—B—303,702 vom 28.1.1975 sind Wachskompositionen bekannt, die dem Bienenwachs ähnliche Emulgier- und Stabilisatoreigenschaften in kometischen Formulierungen aufweisen sollen und die 45—70 Gew.-% einer $\alpha$-alkylverzweigten Monocarbonsäure mit 20—60 Kohlenstoffatomen enthalten. Es hat sich jedoch gezeigt, daß Bienenwachssubstitute mit einem so hohen Anteil an $\alpha$-verzweigten aliphatischen Monocarbonsäuren weder die technischen Kennzahlen des Bienenwachses annähernd aufweisen noch kosmetische Cremes mit ausreichender Konsistenz und Stabilität ergeben.

Es wurde nun gefunden, daß man zu einem Bienenwachsersatz mit dem natürlichen Bienenwachs vergleichbaren Eigenschaften gelangt, wenn diese Komposition aus 3—15 Gew.-% an $\alpha$-verzweigten aliphatischen Monocarbonsäuren der allgemeinen Formel

$$R—CH_2—CH_2—CH(R)—COOH,$$

in der R einen gesättigten, geradkettigen, aliphatischen Kohlenwasserstoffrest mit 12 bis 20 Kohlenstoffatomen darstellt.

15—30 Gew.-% an Estern aus Fettalkohlen der Kettenlängen $C_{12}$—$C_{22}$ und Fettsäuren der Kettenlängen $C_{14}$—$C_{22}$,
5—20 Gew.-% an Triglyceriden der Palmitin-, Stearin-, Hydroxystearin- oder Behensäure,
20—40 Gew.-% an Mono- und/oder Diglyceriden der Palmitin-, Stearin-, Hydroxystearin- oder Behensäure,
5—20 Gew.-% an Fettsäuren bzw. Hydroxyfettsäuren der Kettenlängen $C_{16}$—$C_{22}$,
0—30 Gew.-% an mikrokristallinen Paraffinen des Schmelzbereichs 70—72°C.
besteht.

Unter diesen haben sich fär den erfindungsgemäßen Zweck solche Kompositionen als besonders vorteilhaft erwiesen, die Gemische aus

3—8 Gew.-% an vorgenannten $\alpha$-verzweigten aliphatischen Monocarbonsäuren,
20—30 Gew.-% an Estern aus Fettalkoholen der Kettenlängen $C_{14}$—$C_{20}$ und Fettsäuren der Kettenlängen $C_{16}$—$C_{20}$,
10—15 Gew.-% an Triglyceriden der Palmitin-, Stearin- oder Hydroxystearinsäure,
20—40 Gew.-% an Monoglyceriden der Palmitin-, Stearin- oder Hydroxystearinsäure,
10—20 Gew.-% an Fettsäuren bzw. Hydroxyfettsäuren der Kettenlängen $C_{18}$—$C_{20}$,
10—20 Gew.-% an mikrokristallinen Paraffinen des Schmelzbereichs 70—72°C
darstellen.

Ganz besonders günstige Eigenschaften als Bienenwachsersatz sind einer Komposition aus 5 Gew.-% an $\alpha$-verzweigten aliphatischen Monocarbonsäuren vorgenannter Formel, in der R einen gesättigten, geradkettigen Kohlenwasserstoffrest mit 14 bis 16 Kohlenstoffatomen darstellt,

25 Gew.-% an Cetylpalmitat,
10 Gew.-% an Glycerin-tri-hydroxystearat,
30 Gew.-% an Glycerin-mono-hydroxystearat,
10 Gew.-% an 12-Hydroxystearinsäure,
20 Gew.-% an mikrokristallinem Paraffin vom Schmelzbereich 70—72°C
zuzusprechen.

Diese vorgenannten Kompositionen, insbesondere wiederum die letztgenannte, kommen dem natürlichen Bienenwachs von DAB 7-Qualität sowohl im Hinblick auf die Kenndaten als auch auf das

Verhalten in entsprechenden kosmetischen Präparationen sehr nahe. Als wesentlicher qualitätsbestimmender Bestandteil ist dabei die α-verzweigte aliphatische Monocarbonsäure bzw. das Gemisch der α-verzweigten aliphatischen Monocarbonsäuren anzusehen.

Die für die Herstellung der erfindungsgemäßen Bienenwachsersatz-Kompositionen geeigneten α-verzweigten aliphatischen Monocarbonsäuren lassen sich gemäß DE—A—23 20 461 in vorteilhafter Weise herstellen, indem man a) geradkettige, gesättigte primäre Alkohole der Formel

$$R—CH_2—CH_2—OH,$$

in der R einen Rest mit 12 bis 20, vorzugsweise 14 bis 16 Kohlenstoffatomen darstellt, zu α-verzweigten Alkoholen der Formel

$$R—CH_2—CH_2—\underset{\displaystyle R}{\underset{|}{C}H}—CH_2OH,$$

in der R die vorgenannte Bedeutung hat, in bekannter Weise dimerisiert, b) den erhaltenen α-verzweigten Alkohol durch oxidative Alkalischmelze in das Alkalisalz der entsprechenden α-verzweigten aliphatischen Monocarbonsäure überführt und c) aus dem Alkalisalz durch Ansäuern mit Mineralsäure oder anderen Säuren, die α-verzweigte aliphatische Monocarbonsäure freisetzt. Ausgangsalkohole zur Herstellung der α-verzweigten Monocarbonsäuren stellen Myristylalkohol, Pentadecylalkohol, Cetylalkohol, Stearylalkohol, Nonadecylalkohol, Eicosylalkohol, Behenylalkohol sowie deren Mischungen, wie sie aus natürlichen Fettsäuregemischen gewonnen werden können, dar.

Zur Herstellung der erfindungsgemäßen Bienenwachsersatz-Kompositionen werden die einzelnen Bestandteile unter Vermeidung örtlicher Überhitzungen sorgfältig zusammengeschmolzen. Die so erhaltene Wachsmasse kann an Stelle von Bienenwachs in üblicher Weise in kosmetischen Präparationen wie Hautcremes, Lippenstiften, Cremeemulsionen etc. eingesetzt werden. Weitere in derartigen kosmetischen Zubereitungen übliche Bestandteile wie Emulgatoren, Fettstoffe, Vaseline, Paraffinöl, Glycerin, Vitamine, Pflanzenextrakte, Konservierungsmittel, Farbstoffe lassen sich ohne Schwierigkeiten gemeinsam mit dem erfindungsgemäßen Bienenwachsersatz verarbeiten. Dabei werden Produkte gewonnen, die gegenüber den mit Bienenwachs hergestellten keine nennenswerten Unterschiede aufweisen.

Die nachfolgenden Beispiele sollen den Gegenstand der Erfindung näher erläutern.

## Beispiele

Herstellung der α-verzweigten aliphatischen Monocarbonsäuren

Die Herstellung der als Zwischenprodukte dienenden α-verzweigten Fettalkohole erfolgte nach folgendem Schema:

Ca. 5 Mol des Ausgangsalkohols wurden mit 25 g 50 %iger Kalilauge, 0,2 g Eisen-(III)-sulfat und 0,1 g Kupfersulfat im Stickstoffstrom langsam auf ca. 300°C erhitzt und so lange bei dieser Temperatur gehalten, bis sich kein Wasser mehr abspaltete. Das Rohprodukt wurde alkalifrei gewaschen, getrocknet und vom nicht umgesetzten Ausgangsalkohol durch Destillation befreit. Nach diesem Verfahren wurden folgende α-verzweigte Fettalkohole (Guerbetfettalkohole) hergestellt:

Guerbetfettalkohol C28: Hydroxylzahl 136, Jodzahl 2,5 Ausgangsalkohol n-Tetradecanol-1.

Guerbetfettalkohol C32/36: Hydroxylzahl 106, Jodzahl 5, Schmelzpunkt 34,5°C, Ausgangsalkohol Mischung 1:1 aus n-Hexadecanol-1 und n-Octadecanol-1.

Guerbetalkohol C44: Hydroxylzahl 87, Jodzahl 2,5 Ausgangsalkohol n-Docosanol-1.

Zur Herstellung der entsprechenden α-verzweigten aliphatischen Monocarbonsäure (Guerbetfettsäuren) wurden die Guerbetfettalkohole in Gegenwart von überschüssigem Alkali im Autoklaven auf 300—350°C erhitzt. Durch Zersetzen der entstandenen Natriumsalze mit verdünnter Mineralsäure wurden die freien Guerbetfettsäuren erhalten.

Guerbetfettsäure C28: Säurezahl 130, Verseifungszahl 131,5, Jodzahl 1,98, Tropfpunkt 51°C.
Guerbetfettsäure C32/36: Säurezahl 116, Verseifungszahl 121, Jodzahl 4, Tropfpunkt 63°C.
Guerbetfettsäure C44: Säurezahl 92, Verseifungszahl 93,7, Jodzahl 2,4, Tropfpunkt 65°C.

Zur Herstellung der erfindungsgemäßen Bienenwachsersatz-produkte wurden die vorgenannten Guerbetfettsäuren mit den übrigen Bestandteilen bei ca. 65°C zusammengeschmolzen. Es wurden nachfolgende Kombinationen erstellt.

A) *Kombination mit Guerbetfettsäure C28*

| | |
|---|---|
| Guerbetfettsäure C28 | 15 Gew.-Teile |
| Cetylpalmitat | 30 Gew.-Teile |
| Glycerin-tri-palmitat | 10 Gew.-Teile |
| Glycerin-mono-hydroxystearat | 25 Gew.-Teile |
| 12-Hydroxystearinsäure | 20 Gew.-Teile |

B) *Kombinationen mit Guerbetfettsäure C32/36*

| | Gewichtsteile | | |
|---|---|---|---|
| Guerbetfettsäure C32/36 | 5 | 10 | 5 |
| Cetylpalmitat | 25 | 30 | 30 |
| Glycerin-tri-hydroxystearat | 10 | 10 | 15 |
| Glycerin-mono-hydroxystearat | 30 | 30 | 40 |
| 12-Hydroxystearinsäure | 10 | 20 | 10 |
| Mikrowachs HP 67 | 20 | — | — |

C) *Kombination mit Guerbetfettsäure C44*

| | |
|---|---|
| Guerbetfettsäure C44 | 5 Gew.-Teile |
| Cetylpalmitat | 15 Gew.-Teile |
| Glycerin-tri-palmitat | 20 Gew.-Teile |
| Glycerin-mono-hydroxystearat | 20 Gew.-Teile |
| Palmitinsäure | 10 Gew.-Teile |
| Mikrowachs HP 67 | 30 Gew.-Teile |

Bei der Gegenüberstellung der Kenndaten ergibt sich folgendes Bild:

| | Kombination B mit Mikrowachs | Bienenwachs DAB 7 |
|---|---|---|
| Säurezahl | 22,4 | 17—24 |
| Verseifungszahl | 109 | 83—106 |
| Jodzahl | 4,5 | — |
| Peroxidzahl | 3 | — |
| Steigschmelzpunkt | 64°C | 61—66°C |

Unter Verwendung der Kombination B mit Mikrowachs wurden im Vergleich mit weißem Bienenwachs in üblicher Weise folgende kosmetische Präparationen hergestellt und auf Struktur, Konsistenz und Lagerungsverhalten geprüft. Dabei zeigten sich keinerlei Unterschiede zwischen den Produkten, die den erfindungsgemäßen Bienenwachsersatz enthielten und solchen, die mit weißem Bienenwachs zubereitet waren.

| Wasser-in-Öl-Hautcremes | % Gehalt | |
|---|---|---|
| Gemisch höhermolekularer Ester (Dehymuls E)[(R)] | 7,0 | 7,0 |
| Kombination B mit Mikrowachs | 3,0 | — |
| Bienenwachs, weiß | — | 3,0 |
| Ölsäuredecylester | 6,0 | 6,0 |
| Vaseline, weiß | 12,0 | 12,0 |
| Paraffinöl, dickflüssig | 6,0 | 6,0 |
| Magnesiumsulfat-7-hydrat | 0,3 | 0,3 |
| Glycerin 86 %ig DAB 7 | 5,0 | 5,0 |
| Wasser | 60,7 | 60,7 |
| Struktur und Konsistenz nach 6 Monaten | glatt/weich | glatt/weich |
| Stabilität nach 10 Wochen: | | |
| bei Raumtemperatur | stabil | stabil |
| bei 40°C | stabil | stabil |
| bei —6°C | stabil | stabil |

| Öl-in-Wasser-Emulsionen | % Gehalt | |
|---|---|---|
| Gemisch von Mono- und Diglyceriden der Palmitin- und Stearinsäure (Cutina MD)(R) | 4,0 | 4,0 |
| Stearinsäure | 8,0 | 8,0 |
| Cetylstearylalkohol mit 12 Mol Ethylenoxid | 1,5 | 1,5 |
| Cetylstearylalkohol mit 20 Mol Ethylenoxid | 1,5 | 1,5 |
| 2-Octyldodecanol | 15,0 | 15,0 |
| Kombination B mit Mikrowachs | 2,0 | — |
| Bienenwachs, weiß | — | 2,0 |
| Paraffinöl, dickflüssig | 20,0 | 20,0 |
| Triethanolamin | 0,2 | 0,2 |
| Wasser | 47,8 | 47,8 |
| Struktur und Konsistenz | glatt/weich | glatt/weich |
| Stabilität nach 4 Wochen: | | |
| bei Raumtemperatur | stabil | stabil |
| bei 40°C | stabil | stabil |

| Lippenstifte | Gewichsteile | |
|---|---|---|
| Lippenstiftgrundmasse mit 5 Gew.-% Kombination B mit Mikrowachs | 72 | — |
| Lippenstiftgrundmasse mit 5 Gew.-% Bienenwachs, weiß | — | 72 |
| Capryl-Caprinsäure-triglycerid | 18 | 18 |
| Pigmentfarbe | 4,5 | 4,5 |
| Goldpigment | 5 | 5 |
| Abrieb | gut | gut |
| Steigschmelzpunkt | 70°C | 73°C |
| Stabilität nach 6 Wochen: | | |
| bei Raumtemperatur | keine Ausölung | |
| bei 50°C | keine Ausölung | |

**Patentansprüche**

1. Bienenwachsersatz, bestehend aus einer Komposition von 3—15 Gew.-% an $\alpha$-verzweigten aliphatischen Monocarbonsäuren der allgemeinen Formel

$$R—CH_2—CH_2—\underset{\underset{R}{|}}{CH}—COOH,$$

in der R einen gesättigten, geradkettigen aliphatischen Kohlenwasserstoffrest mit 12—20 Kohlenstoffatomen darstellt,

15—30 Gew.-% an Estern aus Fettalkoholen der Kettenlängen $C_{12}$—$C_{22}$ und Fettsäuren der Kettenlängen $C_{14}$—$C_{22}$,
5—20 Gew.-% an Triglyceriden der Palmitin-, Stearin-, Hydroxystearin-oder Behensäure,
20—40 Gew.-% an Mono- und/oder Diglyceriden der Palmitin-, Stearin-, Hydroxystearin- oder Behensäure,
5—20 Gew.-% an Fettsäuren oder Hydroxyfettsäuren der Kettenlängen $C_{16}$—$C_{20}$,
0—30 Gew.-% an mikrokristallinen Paraffinen des Schmelzbereiches 70—72°C.

2. Bienenwachsersatz nach Anspruch 1, bestehend aus einer Komposition von

3—8 Gew.-% an vorgenannten $\alpha$-verzweigten aliphatischen Monocarbonsäuren,
20—30 Gew.-% an Estern aus Fettalkoholen der Kettenlängen $C_{14}$—$C_{20}$ und Fettsäuren der Kettenlängen $C_{16}$—$C_{20}$,
10—15 Gew.-% an Triglyceriden der Palmitin-, Stearin- oder Hydroxystearinsäure,
20—40 Gew.-% an Monoglyceriden der Palmitin-, Stearin- oder Hydroxystearinsäure,
10—20 Gew.-% an Fettsäuren bzw. Hydroxyfettsäuren der Kettenlängen $C_{18}$—$C_{20}$,
10—20 Gew.-% an mikrokristallinen Paraffinen des Schmelzbereichs 70—72°C.

3. Bienenwachsersatz nach Anspruch 1—2, bestehend aus einer Komposition von

5 Gew.-% an $\alpha$-verzweigten aliphatischen Monocarbonsäuren der allgemeinen Formel

$$R—CH_2—CH_2—\underset{\underset{R}{|}}{CH}—COOH,$$

in der Reinen gesättigten geradkettigen Kohlenwasserstoffrest mit 14—16 Kohlenstoffatomen darstellt,

25 Gew.-% an Cetylpalmitat,
10 Gew.-% an Glycerin-tri-hydroxystearat,
30 Gew.-% an Glycerin-mono-hydroxystearat,
10 Gew.-% an 12-Hydroxystearinsäure,
20 Gew.-% an mikrokristallinem Paraffin vom Schmelzbereich 70—72°C.

4. Verwendung des Bienenwachsersatzes gemäß Anspruch 1 bis 3 in der für Bienenwachs üblichen Art und Menge in kosmetischen Präparaten.

**Revendications**

1. Substitut de cire d'abeilles consistant en une composition de

3 à 15 % en poids d'acides monocarboxyliques aliphatiques à ramification en alpha répondant à la formule générale

$$R—CH_2—CH_2—\underset{\underset{R}{|}}{CH}—COOH,$$

dans laquelle R représente un reste hydrocarboné aliphatique saturé à chaîne droite contenant de 12 à 20 atomes de carbone,
15 à 30 % en poids d'esters d'alcools gras à longeurs de chaîne en C12—C22 et d'acide gras à

longueurs de chaîne en C14—C22,

5 à 20 % en poids de triglycérides de l'acide palmitique, stéarique, hydroxystéarique ou béhénique,

20 à 40 % en poids de mono- et/ou di-glycérides de l'acide palmitique, stéarique, hydroxystéarique ou béhénique,

5 à 20 % en poids d'acides gras ou d'hydroxyacides gras à longueurs de chaîne en C16—C20,

0 à 30 % en poids de paraffine microcristalline fondant dans l'intervalle de 70 à 72°C.

2. Substitut de la cire d'abeilles selon la revendication 1, consistant et une composition de

3 à 8 % en poids des acides monocarboxyliques aliphatiques à ramification en alpha mentionnés ci-dessus,

20 à 30 % en poids d'esters d'alcools gras à longueurs de chaîne en C14—C20 et d'acides gras à longueurs de chaîne en C16—C20,

10 à 15 % en poids de triglycérides de l'acide palmitique, stéarique ou hydroxystéarique,

20 à 40 % en poids de monoglycérides de l'acide palmitique, stéarique ou hydroxystéarique,

10 à 20 % en poids d'acides gras ou d'hydroxyacides gras à longueurs de chaîne en C18—C20,

10 à 20 % en poids de paraffine microcristalline fondant dans l'intervalle de 70 à 72°C.

3. Substitut de la cire d'abeilles selon les revendications 1 et 2, consistant en une composition de

5 % en poids d'acides monocarboxyliques aliphatiques à ramification en alpha de formule générale

$$R-CH_2-CH_2-\underset{\displaystyle R}{\underset{|}{CH}}-COOH,$$

dans laquelle R représente un reste hydrocarboné saturé à chaîne droite contenant de 14 à 16 atomes de carbone,

25 % en poids de palmitate de cétyle,

10 % en poids de tri-hydroxystéarate du glycérol,

30 % en poids de mono-hydroxystéarate du glycérol,

10 % en poids d'acide 12-hydroxystéarique,

20 % en poids d'une paraffine microcristalline fondant dans l'intervalle de 70 à 72°C.

4. Utilisation du substitut de cire d'abeilles selon les revendications 1 à 3 de la manière et aux quantités habituelles pour la cire d'abeilles dans des compositions cosmétiques.

**Claims**

1. A beeswax substitute consisting of a composition of

from 3 to 15% by weight of $\alpha$-branched aliphatic monocarboxylic acids corresponding to the following general formula

$$R-CH_2-CH_2-\underset{\displaystyle R}{\underset{|}{CH}}-COOH,$$

in which R is a saturated, linear $C_{12}$—$C_{20}$ aliphatic hydrocarbon radical,

from 15 to 30% by weight of esters of fatty alcohols having chain lengths of from $C_{12}$ to $C_{22}$ and fatty acids having chain lengths of from $C_{14}$ to $C_{22}$,

from 5 to 20% by weight of triglycerides of palmitic, stearic, hydroxystearic or behenic acid,

from 20 to 40% by weight of mono- and/or diglycerides of palmitic, stearic, hydroxystearic or behenic acid,

from 5 to 20% by weight of fatty acids or hydroxy-fatty acids having chain lengths of from $C_{16}$ to $C_{20}$,

from 0 to 30% by weight of microcrystalline paraffins melting at temperatures in the range from 70 to 72°C.

2. A beeswax substitute as claimed in Claim 1, consisting of a composition of

from 3 to 8% by weight of the above-mentioned $\alpha$-branched aliphatic monocarboxylic acids,

from 20 to 30% by weight of esters of fatty alcohols having chain lengths of from $C_{14}$ to $C_{20}$ and fatty acids having chain lengths of from $C_{16}$ to $C_{20}$,

from 10 to 15% by weight of triglycerides of palmitic, stearic or hydroxystearic acid,

from 20 to 40% by weight of monoglycerides of palmitic stearic or hydroxystearic acid,

from 10 to 20% by weight of fatty acids or hydroxy fatty acids having chain lengths of from $C_{18}$ to $C_{20}$,

from 10 to 20% by weight of microcrystalline paraffins melting at temperatures in the range from 70 to 72°C.

3. A beeswax substitute as claimed in Claims 1 and 2, consisting of a composition of

5% by weight of $\alpha$-branched aliphatic monocarboxylic acids corresponding to the following general formula

$$R—CH_2—CH_2—\underset{\displaystyle R}{\underset{|}{CH}}—COOH,$$

in which R is a saturated, linear $C_{14}$—$C_{16}$ hydrocarbon radical,

25% by weight of cetyl palmitate,
10% by weight of glycerol trihydroxystearate,
30% by weight of glycerol monohydroxystearate,
10% by weight of 12-hydroxystearic acid,
20% by weight of microcrystalline paraffin melting in the range from 70 to 72°C.

4. The use of the beeswax substitute claimed in Claims 1 to 3 in the form and quantity normal for beeswax in cosmetic preparations.